# FASCICULE DE BREVET EUROPEEN

(11) **EP 1 420 008 B1**
(45) Date de publication et mention de la délivrance du brevet: **20.12.2006**
(21) Numéro de dépôt: 03292515.8
(22) Date de dépôt: 10.10.2003
(51) Int. Cl.: C07C 215/08, C07C 217/62, A61K 8/41, A61Q 19/06

(54) **Composition, notamment cosmétique, comprenant une amine secondaire ou tertiaire**
Zusammensetzung, im Besonderen kosmetische, enthaltend ein sekundäres oder tertiäres Amin
Composition, in particular cosmetic, comprising a secondary or tertiary amine

(30) Priorité: 15.11.2002 FR 0214321
(43) Date de publication de la demande: 19.05.2004
(73) Titulaire: L'ORÉAL, 75008 Paris (FR)
(72) Inventeur: Dalko, Maria, 91190 GIF S/Yvette (FR); Breton, Lionel, 78000 Versailles (FR); Boulle, Christophe, Lagny S/Marne 77400 (FR)
(74) Mandataire: Kromer, Christophe

(56) Documents cités:
- EP-A- 0 691 327
- EP-A- 1 090 630
- WO-A-97/37967
- FR-A- 2 798 590
- SHUKER A J ET AL: "The Application of High-Throughput Synthesis And Purification To The Preparation Of Ethanolamines" TETRAHEDRON LETTERS, ELSEVIER SCIENCE PUBLISHERS, AMSTERDAM, NL, vol. 38, no. 35, 1 septembre 1997 (1997-09-01), pages 6149-6152, XP004086721 ISSN: 0040-4039

## Description

La présente invention concerne de nouvelles amines secondaires ou tertiaires, ainsi qu'une composition, adaptée à une application topique sur la peau, comprenant au moins une telle amine dans un milieu physiologiquement acceptable.

Elle concerne également l'utilisation d'une telle amine dans une composition adaptée à une application topique sur la peau, comme agent destiné à lisser les rides et ridules, en particulier les rides et ridules d'expression.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse, ou en prévenant la dégradation, des fibres de soutien qui composent le tissu cutané.

Bien que ces traitements permettent d'agir sur les rides et ridules dues au vieillissement chronologique ou intrinsèque, ainsi que sur celles dues au photo-vieillissement, ils n'ont pas d'effet sur les rides et ridules d'expression, lesquelles nécessitent une intervention sur la composante contractile musculaire des rides présente dans la peau.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21). Les dermatologues ont aussi parfois recours à des implants d'acide hyaluronique ou d'acide polylactique.

Ces techniques ont toutefois l'inconvénient de nécessiter le recours à un praticien.

Dans l'optique de proposer des solutions plus simples d'utilisation que ces techniques médicales, la Demanderesse a proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'Iris pallida (FR-2 746 641).

Il reste toutefois le besoin de disposer de composés efficaces pour lisser ou estomper les rides et ridules d'expression.

Or, la Demanderesse a découvert avec étonnement que certaines amines secondaires et tertiaires permettaient de satisfaire ce besoin.

On connaît, certes, du document EP-1 090 630 des amines secondaires et tertiaires qui ont la propriété d'augmenter la synthèse de collagène par les fibroblastes et d'hydrater la peau et sont de ce fait utiles contre la peau sèche et la dermatite atopique, mais aussi contre les rides. De manière analogue, le document EP-0 691 327 divulgue une famille très vaste d'amines mono-, di- ou trisubstituées décrites comme efficaces pour lisser les rides. Il n'est pas suggéré que les amines divulguées dans ces documents puissent avoir un quelconque effet sur les rides et ridules d'expression, au contraire des amines objet de la présente invention qui constituent une sélection parmi la très vaste famille d'amines divulguée dans EP-1 090 630 et EP-0 691 327.

On connaît par ailleurs du document WO 93/05763 certaines amines di- et trisubstituées par au moins deux chaînes portant chacune au moins un groupe hydroxy. Ces amines augmentent la différenciation des kératinocytes, limitent l'épaississement UV-induit de l'épiderme et sont utiles pour prévenir et traiter les rides induites par le rayonnement UVB. Il n'est pas suggéré que ces amines, différentes de celles objet de la présente invention en ce sens qu'elles ne comprennent pas de groupement phényle, aient un quelconque effet sur les rides et ridules d'expression.

Enfin, on connaît de WO 97/37967 une famille d'antagonistes calciques constitués d'arylalkylamines disubstituées, qui sont notamment capables de moduler la différenciation des kératinocytes. On connaît aussi de la publication de SHUKER A. J. et al, The Application of High-Throughput Synthesis And Purification To The Preparation Of Ethanolamines, *Tetrahedron Letters*, Vol. 38, No. 35, pp. 6149-6152 (1997) une famille d'éthanolamines de structure proche des précédentes, utiles comme modulateurs de récepteurs adrénergiques. Dans ces deux familles de composés, le groupe phényle est séparé de l'atome d'azote par une chaîne ramifiée, ce qui les différencie de ceux objet de la présente invention. En outre, il n'est pas suggéré que ces composés aient un quelconque effet sur les rides, *a fortiori* sur les rides d'expression.

La Demanderesse a maintenant découvert qu'en sélectionnant certaines amines secondaires et tertiaires de structure simple, il était possible d'obtenir des compositions cosmétiques efficaces pour lisser les rides et ridules d'expression.

Il a, certes, été décrit précédemment par la Demanderesse l'utilisation de l'alvérine, qui est une amine trisubstituée, comme agent myorelaxant destiné à lisser les rides d'expression. Toutefois, à la différence des composés objet de la présente invention, l'alvérine est une amine substituée par deux chaînes aralkyles. Or, il n'était pas évident que l'activité myorelaxante de l'alvérine soit augmentée par substitution dans sa molécule d'une chaîne alkyle à une chaîne aralkyle.

La présente invention a donc pour objet de nouvelles amines secondaires ou tertiaires de formule (I) : 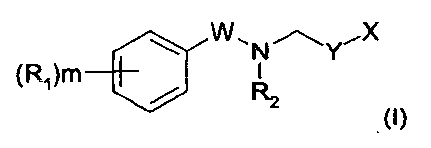 dans laquelle :
R₁ désigne un groupe alkyle en C₁-C₈. linéaire ou ramifié, saturé ou insaturé, ou un groupe -CN, -OR₁₁, -SR₁₁, -NR₁₁R₁₂, -COR₁₁, -COOR₁₁, -CONR₁₁R₁₂, -NR₁₁-CO-R₁₂, -NR₁₁-CO-NR₁₂R₁₃ ou -CF₃ ou un atome d'halogène,
   où R₁₁, R₁₂ et R₁₃ désignent indépendamment un atome d'hydrogène ou un groupe alkyle en C₁-C₄, linéaire ou ramifié, ou un groupe aryle éventuellement substitué par un groupe -OR, -NRR', -CCOR ou CF₃,
   où R et R' désignent indépendamment un atome d'hydrogène ou un groupe alkyle en C₁-C₄, linéaire ou ramifié,
R₂ désigne un atome d'hydrogène ou un groupe alkyle linéaire ou ramifié, saturé ou insaturé, en C₁-C₁₂, non substitué,
W est une chaîne alkylène ou alkénylène linéaire, non substituée, en C₂-C₄,
X est un groupe -OR₁₁ ou -NR₁₁R₁₂, où R₁₁ et R₁₂ ont la signification indiquée ci-dessus,
Y désigne une chaîne di (C₅-C₇)alkyl éthylène ou di (C₅-C₇)alkyl propylène,
m est un entier compris entre 0 et 5,
   étant entendu que, lorsque m est non nul, les groupes R₁ peuvent être identiques ou différents,
ou son sel d'addition avec un acide ou ses isomères ou stéréo-isomères.

Dans la formule (I), les groupes alkyle peuvent être choisis, selon le cas, parmi les groupes : méthyle, éthyle, n-propyle, isopropyle, n-butyle, isobutyle, tert-butyle, pentyle, hexyle, heptyle, octyle, nonyle, décyle, undécyle et dodécyle.

De son côté, le groupe aryle peut être un groupe phényle.

La chaîne alkylène ou alkénylène en C₂-C₄ est en particulier une chaîne éthylène, triméthylène, tétraméthylène, vinylène, ou propénylène.

Y désigne de préférence une chaîne dipentyléthylène.

L'atome d'halogène peut être un atome de fluor, de chlore ou de brome.

Comme sels du composé de formule (I), on peut citer les sels obtenus par addition du composé de formule (I) avec un acide inorganique, choisi notamment parmi les acides chlorhydrique, sulfurique, nitrique et phosphorique, ou avec un acide organique, choisi en particulier parmi les acides succinique, fumarique, lactique, glycolique, citrique et tartrique.

Selon une forme d'exécution préférée de l'invention, le composé de formule (I) est tel que l'une au moins des conditions suivantes est satisfaite :
- m = 0 ou 1,
- R₁ est un groupe -OR₁₁ où R₁₁ est un atome d'hydrogène ou un groupe alkyle en C₁-C₄, linéaire ou ramifié,
- R₂ est un atome d'hydrogène ou un groupe alkyle en C₁-C₆, linéaire ou ramifié, saturé, non substitué,
- W est une chaîne alkylène ou alkénylène, linéaire, non substituée,en C₂-C₄,
- X est un groupe -OR₁₁ où R₁₁ est un atome d'hydrogène ou un groupe alkyle en C₁-C₄, linéaire ou ramifié,
- Y est une chaîne di (C₅-C₇)alkyl éthylène.

De façon plus préférentielle encore, le composé de formule (I) est tel que :
- m = 0 ou 1,
- R₁ est un groupe -OH ou -OCH₃,
- R₂ est un atome d'hydrogène ou un groupe éthyle,
- W est une chaîne triméthylène ou propénylène,
- X est un groupe -OH,
- Y est une chaîne di (C₅-C₇)alkyl éthylène ou di (C₅-C₇)alkyl propylène.

Selon une forme d'exécution particulièrement préférée de l'invention, le composé de formule (I) est tel que : m = 0 ; R₂ est un groupe éthyle ; X = OH ; Y est une chaîne dipentyléthylène ; et W est une chaîne triméthylène.

Les amines de formule (I) peuvent être préparées selon des procédés analogues aux schémas réactionnels présentés sur les figures 1 à 3 annexées et à ceux décrits dans les Exemples 1 à 9 ci-après.

L'invention concerne également une composition, adaptée à une application topique sur la peau, comprenant, dans un milieu physiologiquement acceptable, au moins un composé de formule (I') : 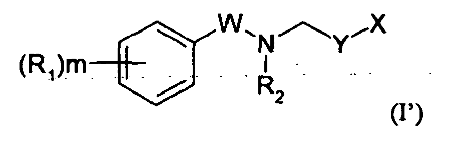 dans laquelle :
R₁ désigne un groupe alkyle en C₁-C₈, linéaire ou ramifié, saturé ou insaturé, ou un groupe -CN, -OR₁₁, -SR₁₁, -NR₁₁R₁₂, -COR₁₁, -COOR₁₁, -CONR₁₁R₁₂, -NR₁₁-CO-R₁₂, -NR₁₁-CO-NR₁₂R₁₃ ou -CF₃ ou un atome d'halogène,
   où R₁₁, R₁₂ et R₁₃ désignent indépendamment un atome d'hydrogène ou un groupe alkyle en C₁-C₄, linéaire ou ramifié, ou un groupe aryle éventuellement substitué par un groupe -OR, -NRR', -COOR ou CF₃,
   où R et R' désignent indépendamment un atome d'hydrogène ou un groupe alkyle en C₁-C₄, linéaire ou ramifié,
R₂ désigne un atome d'hydrogène ou un groupe alkyle linéaire ou ramifié, saturé ou insaturé, en C₁-C₁₂, non substitué,
W est une chaîne alkylène ou alkénylène en C₂-C₄ linéaire, non substituée, ou une chaine méthylène,
X est un groupe -OR₁₁ ou -NR₁₁R₁₂, où R₁₁ et R₁₂ ont la signification indiquée ci-dessus,
Y désigne une chaîne alkylène ou alkénylène en C₁₁-C₂₀, linéaire ou ramifiée, non substituée,
m est un entier compris entre 0 et 5,
   étant entendu que, lorsque m est non nul, les groupes R₁ peuvent être identiques ou différents,
ou un sel d'addition avec un acide ou un isomère ou stéréo-isomère de ce composé.

La quantité d'amine de formule (I') utilisable selon l'invention est bien entendu fonction de l'effet recherché et peut donc varier dans une large mesure. Pour donner un ordre de grandeur, on peut utiliser cette amine en une quantité représentant de 0,01% à 10% du poids total de la composition, préférentiellement en une quantité représentant de 0,05% à 5% du poids total de la composition, plus préférentiellement en une quantité représentant de 0,1% à 2% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses.

Cette composition peut se présenter sous toutes les formes galéniques normalement utilisées dans le domaine cosmétique, et elle peut être notamment sous forme d'une solution huileuse éventuellement gélifiée, d'une dispersion du type lotion biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des amines de formule (I) selon invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; d'autres agents myorelaxants ; les agents tenseurs ; les agents anti-pollution et/ou anti-radicalaire ; les agents agissant sur la microcirculation ; les agents agissant sur le métabolisme énergétique des cellules ; et leurs mélanges.

La composition selon l'invention peut également renfermer des filtres UVA et/ou UVB, sous forme de composés organiques ou inorganiques, ces derniers étant éventuellement enrobés pour les rendre hydrophobes.

Les agents photoprotecteurs organiques plus particulièrement préférés sont choisis parmi les composés suivants (identifiés sous leur nom CTFA) : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetrasulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques peuvent être choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium.

Comme cela sera démontré dans les Exemples ci-après, la Demanderesse a mis en évidence un effet myorelaxant des amines de formule (I') selon l'invention, qui permet d'envisager leur utilisation, plus particulièrement dans le lissage des rides et ridules d'expression.

L'invention a donc aussi pour objet l'utilisation cosmétique d'au moins une amine de formule (I') telle que définie ci-dessus, dans une composition adaptée à une application topique sur la peau, comme agent destiné à lisser les rides et ridules, en particulier d'expression.

Elle a encore pour objet un procédé de traitement cosmétique d'une peau ridée, comprenant l'application topique sur ladite peau d'une composition telle que définie précédemment dans laquelle W peut en outre désigner une chaîne méthylène.

La composition selon l'invention est avantageusement destinée à être appliquée sur les zones du visage ou du front marquées par des rides et ridules d'expression, et/ou sur les personnes présentant des rides et ridules d'expression.

Les rides et ridules concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

L'invention sera maintenant illustrée par les exemples non limitatifs suivants.

### EXEMPLES

### Exemple 1 : synthèse du 6-[2-{ethyl-(3-phenyl-propyl)amino}-ethyl]-undecan-6-ol (composé 1)

Ce composé est préparé selon le schéma réactionnel présenté à la Figure 1.

### a) Ethyl-(3-phenyl-propyl)-amine

1 équivalent de 3-phénylpropylamine (36,98 mmol ; 5,3 ml) commerciale, 1 équivalent d'acétaldéhyde (36,98 mmol ; 2,1 ml) et 25 ml d'éthanol sont mélangés et chauffés à 50°C pendant 3 heures. On ajoute ensuite 1,4 équivalent de borohydrure de sodium (9,320 mmol ; 353 mg) par petites spatules et on agite à température ambiante pendant 2 heures. Le milieu réactionnel est concentré puis repris dans de l'eau ; on extrait la phase organique avec 2 fractions de 20 ml de dichlorométhane, puis on sèche la phase organique sur sulfate de sodium anhydre et on concentre à sec.

On obtient ainsi 6,0 g de composé **10** sous forme d'une huile jaune (rendement : 99%).

### b) 3-[ethyl-(3-phenyl-propyl)-amino]-propioniate d'éthyle

A 1 équivalent d'ethyl-(3-phenyl-propyl)-amine (2,450 mmol ;400 mg) solubilisé dans 10 ml de diméthylformamide, on ajoute 1,5 équivalent de carbonate de potassium (3,675 mmol ; 507 mg) et 2 équivalents de 3-bromopropionate d'éthyle (4,900 mmol ; 0,57 ml), puis on chauffe à 50°C sous agitation pendant 10 heures. Le milieu réactionnel est dilué dans un grand volume de dichlorométhane, lavé avec une solution saturée en hydrogénocarbonate de sodium, séché sur sulfate de sodium anhydre, filtré et concentré à sec. L'huile obtenue est purifiée sur colonne de silice (dichlorométhane / méthanol).

On obtient 100 mg composé **11** sous forme d'une huile jaune (rendement = 17 %).

### c) 6-[2-(3-phenyl-propylamino)-ethyl]-undecan-6-ol

Dans un montage préalablement séché et sous atmosphère inerte, on verse 2 équivalents de copeaux de magnésium (15,2 mmol ; 370 mg) et on les recouvre d'éther anhydre. On ajoute un cristal d'iode puis quelques gouttes de bromopentane; on agite légèrement et on chauffe. Lorsque la réaction démarre, on ajoute le reste de bromopentane (2 équivalents ; 15,2 mmol ; 1,9 ml) dilué dans de l'éther anhydre. On laisse agiter à température ambiante pendant 1 heure. Quand tout le magnésium s'est dissous, le milieu réactionnel est refroidi à 0 °C, et on ajoute goutte à goutte 1 équivalent de 3-[ethyl-(3-phenyl-propyl)-amino]-propionate d'éthyle (7,6 mmol ; 2 g) dilué dans de l'éther anhydre. On laisse le milieu réactionnel revenir à température ambiante et on agite pendant 15 minutes. On ajoute de l'eau au milieu réactionnel, puis une solution de chlorure d'ammonium à 0,1 M. On extrait la phase organique avec du dichlorométhane, on la lave par une solution de NaHCO₃ à 0,1 M, on la sèche sur sulfate de sodium anhydre et on la concentre à sec. Le produit est purifié sur colonne de silice (2 % méthanol / 98 % dichlorométhane).

On obtient 200 mg de composé 1 sous forme d'une huile jaune (rendement = 8 %).

### Exemple 2 : Synthèse du 6-{2-(3-phenyl-propyl)amino-ethyl}-undecan-6-ol (composé 2)

Ce composé est synthétisé selon le schéma réactionnel présenté à la Figure 2.

A 1 équivalent de 6-(2-amino-ethyl)-undecan-6-ol (6,657 mmol ; 1,43 g) **12** solubilisé dans 5 ml d'éthanol est ajouté goutte à goutte 1 équivalent de 3-phénylpropionaldéhyde (6,657 mmol ; 0,88 ml). Après agitation à température ambiante pendant 3 heures, 1,4 équivalent de borohydrure de sodium (9,320 mmol ; 353 mg) est ajouté par petites spatules et le milieu de nouveau agité à température ambiante pendant 2 heures. Le milieu réactionnel est mis à sec et l'huile obtenue est reprise dans de l'eau. La phase organique est extraite avec 2 fractions de 20 ml de dichlorométhane, séchée sur sulfate de sodium anhydre et concentrée à sec.

On obtient 1,8 g de composé 2 sous forme d'une huile jaune (rendement = 81 %).

### Exemple 3 : Synthèse du 6-(2-{Ethyl-[3-(4-methoxy-phenyl)-propyl]-amino}-ethyl}-undecan-6-ol (composé 3)

Ce composé est préparé selon un schéma réactionnel analogue à celui présenté à la Figure 1.

On prépare ainsi l'éthyl-(3-p-methoxy-phenyl-propyl)-amine) à partir de 3-p-methoxy-phenyl-propyl)-amine de manière analogue au composé **10** et le 3-[ethyl-(3-p-methoxy phenyl-propyl)-amino]-propionate d'éthyle de manière analogue au composé **11**. On soumet ensuite l'ester obtenu aux mêmes réactions que le composé **11** pour obtenir le composé 3.

On obtient 250 mg de composé 3 sous forme d'une huile jaune (rendement : 8%).

### Exemple 4 : Synthèse du 6-{2-[3-(4-methoxy-phenyl)-propylamino]-ethyl}-undecan-6-ol (composé 4)

Ce composé est préparé selon un schéma réactionnel analogue à celui présenté à la Figure 2, excepté que le 3-(4-methoxyphényl) propionaldéhyde est substitué au 3-phénylpropionaldéhyde.

On obtient ainsi 1,9 g de composé 4 sous forme d'une huile jaune (rendement : 79%)/

### Exemple 5 : Synthèse du 6-{3-[ethyl-(3-phenyl-propyl)-amino]-propyl}-undecan-6-ol (composé 5)

On prépare le composé 5 de façon analogue au composé 1, excepté que le 3-bromopropionate d'éthyle est remplacé par du 4-bromobutyrate de méthyle.

On obtient 200 mg de composé 5 sous forme d'une huile jaune (rendement : 7%).

### Exemple 6 : Synthèse du 6-[3-(3-phenyl-propylammo)-propyl]-undecan-6-ol (composé 6)

On prépare le composé 6 de façon analogue au composé 2, excepté que le réactif **12** est remplacé par le 6-(3-amino-propyl)undecan-6-ol.

On obtient 1,9 g de composé 6 sous forme d'une huile (rendement : 82%).

### Exemple 7 : Synthèse du 6-(3-{Ethyl-[3-(4-methoxy-phenyl)-propyl]-amino}-propyl}-undecan-6-ol (composé 7)

On prépare le composé 7 de façon analogue au composé 1, en utilisant comme produit de départ la 3-(4-méthoxy)phényl propylamine et en remplaçant le 3-bromopropionate d'éthyle par le 3-bromobutyrate d'éthyle.

On obtient 230 mg de composé 7 sous forme d'une huile jaune (rendement : 7%).

### Exemple 8 : Synthèse du 6-{3-[3-(4-methoxy-phenyl)-propylamino]-propyl}-undecan-6-ol (composé 8)

Le composé 8 est préparé de façon analogue au composé 4, excepté que l'aldéhyde de départ est mis à réagir avec le 6-(3-amino-propyl)-undecan-6-ol.

On obtient 1,9 g de composé 8 sous forme d'une huile (rendement : 76%).

### Exemple 9 : Synthèse du 6-{2-[ethyl-(3-phenyl-allyl)-amîno]-ethyl}-undecan-S-öl (composé 9)

Ce composé est préparé selon le schéma réactionnel illustré à la Figure 3.

### a) Ethyl-(3-phenyl-allyl)-amine

A 1 équivalent de cinnamaldéhyde (0,038 mmol ; 5 g) mélangé avec 10 ml d'éthanol est ajouté goutte à goutte 1 équivalent d'éthylamine à 70 % dans l'eau (0,038 mmol ; 2,44 g). On agite à température ambiante pendant 1 heure, puis on ajoute 2 équivalents de borohydrure de sodium (0,076 mmol ; 2,86 g) par petites spatules (réaction exothermique) et on laisse agiter à température ambiante pendant 1 heure. On ajoute alors une grande quantité d'eau et on extrait la phase organique avec du dichlorométhane, on la sèche sur sulfate de sodium anhydre et on la concentre à sec.

On obtient 5,51 g de composé **13** sous forme d'une huile jaune (rendement : 90%).

### b) 3-[ethyl-(3-phenyl-allyl)-amino]-propionate d'éthyle

Dans un tricol de 50 ml sont mélangés 1 équivalent d'éthyl-(3-phenyl-allyl)-amine **13** (0,031 mmol ; 5 g), 1 équivalent de carbonate de potassium (0,031 mmol ; 4,3 g) et 25 ml de diméthylformamide. On ajoute alors goutte à goutte 1 équivalent de 3-bromopropionate d'éthyle (0,031 mmol ; 3,4 ml) et on agite à température ambiante pendant 5 heures. Le milieu réactionnel est dilué dans du dichlorométhane et lavé avec une solution saturée en hydrogénocarbonate de sodium. La phase organique est séchée sur sulfate de sodium anhydre et concentrée à sec.

On obtient 6,5 g de composé **14** sous forme d'une huile jaune (rendement : 80%).

### c) 6-{2-[ethyl-(3-phenyl-allyl)-amino]-ethyl}-undecan-6-ol

Au montage préalablement séché, on ajoute 2 équivalents de copeaux de magnésium (0,023 mmol ; 560 mg) et on recouvre ces derniers d'éther anhydre. On ajoute un cristal d'iode et quelques gouttes de bromopentane pur ; on agite légèrement et on chauffe un peu si nécessaire. Lorsque la réaction est démarrée, on ajoute le reste de bromopentane (2 équivalents ; 0,023 mmol ; 2,86 ml) dilué dans de l'éther anhydre (réaction exothermique). On laisse agiter à température ambiante pendant 1 heure environ. Quand tout le magnésium s'est dissous, on refroidit le milieu réactionnel à 0 °C, et on ajoute goutte à goutte 1 équivalent de 3-[ethyl-(3-phenyl-allyl)-amino]-propionate d'éthyle (0,012 mmol ; 3 g) **14** dilué dans de l'éther anhydre ; on laisse le milieu réactionnel revenir à température ambiante et on agite pendant 1 quart d'heure. On ajoute de l'eau au milieu réactionnel, puis une solution de chlorure d'ammonium à 0,1 M. On extrait la phase organique avec du dichlorométhane, on la sèche sur sulfate de sodium anhydre et on la concentre à sec. Le produit est purifié sur colonne de silice (dichlorométhane/méthanol).

On obtient 227 mg de composé 9 sous forme d'une huile jaune (rendement : 6%).

### Exemple 10 : Mise en évidence de l'effet myorelaxant des amines selon l'invention

### a) Modèle de jonction nerf / muscle

Le composé 1 a été testé sur un modèle de jonction nerf / muscle (plaque motrice) obtenu dans une préparation nerf phrénique / diaphragme isolé de rat (muscle strié) (Pollard B.J. et al, Br.J. Anaesth., 1988, 61, 419-424).

Ce test est prédictif d'un effet anti-rides, comme cela a été vérifié par la Demanderesse sur le diazépam, qui est à la fois actif in vivo chez l'homme et dans ce test à 10⁻⁴ M.

Le nerf phrénique et le diaphragme sont soigneusement isolés et placés dans une cuve de 50 ml remplie de liquide de survie (liquide de Krebs Henseleit) maintenu à une température de 37°C et oxygéné à l'aide d'un mélange oxygène/CO₂ (95/5).

Les variations de tension du diaphragme sont enregistrées avec une pré-charge initiale de plusieurs grammes.

Après une période de relaxation de 30 mn, le diaphragme est stimulé indirectement par l'intermédiaire du nerf phrénique.

Sur chaque préparation, l'effet du composé à tester a été évalué sur les contractions induites par stimulation indirecte via stimulation sur le nerf phrénique (0.1 à 0.5 volts, 0.3 ms, 0.1 Hz). L'alvérine, connue comme composé myorelaxant à effet anti-rides d'après la demande FR-2 798 590, est utilisée comme témoin.

Les résultats obtenus sont présentés dans le tableau ci-dessous :

| PRODUIT | CONCENTRATION | % INHIBITION |
|---|---|---|
| | | DES |
| | | CONTRACTIONS |
| Alvérine | 10⁻⁴ M | 100% |
| Composé 1 | 10⁻⁴ M | 100% |

### b) Test sur canaux calciques

Le test mesure la capacité d'un produit à inhiber par compétition la fixation d'agonistes de canaux calciques de type L, type Vérapamil. Ces canaux ont été identifiés au niveau des fibroblastes humains (Baumgarten LB and coll (1992), *J. Biol. Chem,* 267, 10524-10530 et Chen CF and coll (1988), *Science,* 239, 1024-1026).

Les études sont réalisées à partir d'homogénats de cortex cérébral de rat (membranes isolées présentant à leur surface notamment les canaux calciques de type L).

Les conditions expérimentales selon le protocole décrit par Reynolds I.J. et al., 1986, *J. Pharmacol. Exp. Ther.,* 237, p.731, sont présentées dans le tableau 1 ci-dessous :

**Tableau 1**

| Test | Ligand | Conc. | Non spécifique | Incubation | Détection |
|---|---|---|---|---|---|
| Canal Ca²⁺ (L, site verapamil) | [³H](-) D 888 | 0.5 nM | D 600 (10 µM) | 60 min. /22°C | Comptage scintillation |

D888 et D600 sont les molécules de référence spécifiques des canaux calciques de type L, site vérapamil.

La liaison spécifique d'un ligand (D888 marqué) aux récepteurs (canaux calciques de type L, site vérapamil) est défini comme la différence entre la liaison totale et la liaison non spécifique déterminée en présence d'un excès de ligand non radioactif. Les résultats sont exprimés en pourcentage de liaison spécifique témoin et en pourcentage d'inhibition de cette liaison en présence des composés testés.

Les valeurs d'IC₅₀ (concentration inhibant 50% de la liaison spécifique témoin) et du coefficient de Hill (nH) sont calculées pour les composés testés à partir des courbes de compétition selon un modèle de régression non linéaire. Ces paramètres sont obtenus par "curve-fitting" de l'équation de Hill.

Deux composés ont été testés : l'alvérine, qui est un agent myorelaxant inhibiteur de canaux calciques d'après la demande FR-2 798 590, et le composé 1 décrit précédemment (Exemple 1), chacun aux concentrations de 1 µM et 100 µM. Les mesures sont effectuées en double. La molécule de référence (D600) est parallèlement testée à huit concentrations et en double pour obtenir une courbe de compétition permettant de valider ce test.

Les résultats obtenus sont reportés dans le Tableau 2 ci-dessous :

**Tableau 2**

| Composé | IC₅₀ |
|---|---|
| Alvérine | 693 nM |
| Composé 1 | 325 nM |

Le composé 1 selon l'invention est donc un meilleur inhibiteur de canaux calciques que l'alvérine.

### c) Conclusion

Il ressort des deux tests ci-dessus que les composés selon l'invention sont des agents myorelaxants au moins aussi efficaces que l'alvérine, voire davantage, et utiles à cet effet dans le lissage des rides et ridules d'expression.

### Exemple 11 : Composition cosmétique

Cette composition est préparée de manière classique pour l'homme du métier. Les quantités indiquées sont en pourcentages pondéraux.

| | |
|---|---|
| Composé 1 | 1 % |
| Isostéarate de propylène glycol | 13 % |
| Polyéthylène glycol (8 OE) | 5 % |
| Propylène glycol | 3 % |
| Pentylène glycol | 3 % |
| Stéarate de glycéryle et stéarate de polyéthylène glycol (100 OE) | 5 % |
| Mono-stéarate de sorbitane oxyéthyléné (20 OE) | 0,5 % |
| Alcool cétylique oxyéthyléné (20 OE) oxypropyléné (5 OP) | 1 % |
| Gélifiants | 0,5 % |
| Benzoates d'alkyle en C₁₂₋₁₅ | 4 % |
| Ethanol | 3 % |
| Hydroxyde de sodium | 0,12 % |
| Conservateurs | 0,7 % |
| Eau | qsp 100 % |

Ce fluide est destiné à être utilisé en applications mono- ou biquotidiennes sur le visage et le front pour atténuer les rides et ridules d'expression et détendre les traits du visage.

## Revendications

1. Composé de formule (I) : 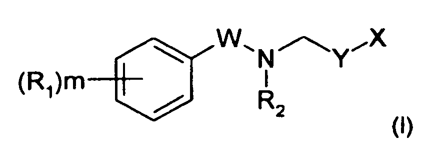 dans laquelle :
R₁ désigne un groupe alkyle en C₁-C₈, linéaire ou ramifié, saturé ou insaturé, ou un groupe -CN, -OR₁₁, -SR₁₁, -NR₁₁R₁₂, -COR₁₁, -COOR₁₁, -CONR₁₁R₁₂, -NR₁₁-CO-R₁₂, -NR₁₁-CO-NR₁₂R₁₃ ou -CF₃ ou un atome d'halogène,
où R₁₁, R₁₂ et R₁₃ désignent indépendamment un atome d'hydrogène ou un groupe alkyle en C₁-C₄, linéaire ou ramifié, ou un groupe aryle éventuellement substitué par un groupe -OR, -NRR', -COOR ou CF₃,
où R et R' désignent indépendamment un atome d'hydrogène ou un groupe alkyle en C₁-C₄, linéaire ou ramifié,
R₂ désigne un atome d'hydrogène ou un groupe alkyle linéaire ou ramifié, saturé ou insaturé, en C₁-C₁₂, non substitué,
W est une chaîne alkylène ou alkénylène en C₂-C₄ linéaire, non substituée,
X est un groupe -OR₁₁ ou -NR₁₁R₁₂, où R₁₁ et R₁₂ ont la signification indiquée ci-dessus,
Y désigne une chaîne di (C₅-C₇)alkyl éthylène ou di (C₅-C₇)alkyl propylène,
m est un entier compris entre 0 et 5,
étant entendu que, lorsque m est non nul, les groupes R₁ peuvent être identiques ou différents,
ou son sel d'addition avec un acide ou ses isomères ou stéréo-isomères.

2. Composé selon la revendication 1, **caractérisée en ce que** le sel est obtenu par addition avec un acide inorganique choisi parmi les acides chlorhydrique, sulfurique, nitrique et phosphorique.

3. Composé selon la revendication 1, **caractérisée en ce que** le sel est obtenu par addition avec un acide organique choisi parmi les acides succinique, fumarique, lactique, glycolique, citrique et tartrique.

4. Composé selon l'une quelconque des revendications 1 à 3, **caractérisée en ce qu'**il est tel que l'une au moins des conditions suivantes est satisfaite :
• m = 0 ou 1,
• R₁ est un groupe -OR₁₁ où R₁₁ est un atome d'hydrogène ou un groupe alkyle en C₁-C₄, linéaire ou ramifié,
• R₂ est un atome d'hydrogène ou un groupe alkyle en C₁-C₆, linéaire ou ramifié, saturé,
• W est une chaîne alkylène ou alkénylène en C₂-C₄, linéaire, non substituée,
• X est un groupe -OR₁₁ où R₁₁ est un atome d'hydrogène ou un groupe alkyle en C₁-C₄, linéaire ou ramifié,
• Y est une chaîne di (C₅-C₇)alkyl éthylène.

5. Composé selon l'une quelconque des revendications 1 à 3, **caractérisée par le fait que** :
• m = 0 ou 1,
• R₁ est un groupe -OH ou -OCH₃,
• R₂ est un atome d'hydrogène ou un groupe éthyle,
• W est une chaîne triméthylène ou propénylène,
• X est un groupe -OH,
• Y est une chaîne di (C₅-C₇)alkyl éthylène ou di (C₅-C₇)alkyl propylène.

6. Composé selon la revendication 5, **caractérisé en ce qu'**il est tel que : m = 0 ; R₂ est un groupe éthyle ; X = OH ; Y est une chaîne dipentyléthylène ; et W est une chaîne triméthylène.

7. Composition, adaptée à une application topique sur la peau, comprenant, dans un milieu physiologiquement acceptable, au moins un composé de formule (I') : 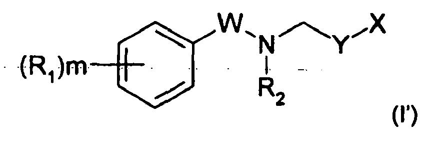 dans laquelle :
R₁ désigne un groupe alkyle en C₁-C₈, linéaire ou ramifié, saturé ou insaturé, ou un groupe -CN, -OR₁₁, -SR₁₁, -NR₁₁R₁₂, -COR₁₁, -COOR₁₁, -CONR₁₁R₁₂, -NR₁₁-CO-R₁₂, -NR₁₁-CO-NR₁₂R₁₃ ou -CF₃ ou un atome d'halogène,
où R₁₁, R₁₂ et R₁₃ désignent indépendamment un atome d'hydrogène ou un groupe alkyle en C₁-C₄, linéaire ou ramifié, ou un groupe aryle éventuellement substitué par un groupe -OR, -NRR', -COOR ou CF₃,
où R et R' désignent indépendamment un atome d'hydrogène ou un groupe alkyle en C₁-C₄, linéaire ou ramifié,
R₂ désigne un atome d'hydrogène ou un groupe alkyle linéaire ou ramifié, saturé ou insaturé, en C₁-C₁₂, non substitué,
W est une chaîne alkylène ou alkénylène en C₂-C₄ linéaire, non substituée ou une chaîne méthylène,
X est un groupe -OR₁₁ ou -NR₁₁R₁₂, où R₁₁ et R₁₂ ont la signification indiquée ci-dessus,
Y désigne une chaîne alkylène ou alkénylène en C₁₁-C₂₀, linéaire ou ramifiée, non substituée,
m est un entier compris entre 0 et 5,
étant entendu que, lorsque m est non nul, les groupes R₁ peuvent être identiques ou différents,
ou un sel d'addition avec un acide ou un isomère ou stéréo-isomère de ce composé.

8. Composition selon la revendication 7, **caractérisée en ce que** le sel est obtenu par addition avec un acide inorganique choisi parmi les acides chlorhydrique, sulfurique, nitrique et phosphorique.

9. Composition selon la revendication 7, **caractérisée en ce que** le sel est obtenu par addition avec un acide organique choisi parmi les acides succinique, fumarique, lactique, glycolique, citrique et tartrique.

10. Composition selon l'une quelconque des revendications 7 à 9, **caractérisée en ce que** le composé de formule (I') est tel que l'une au moins des conditions suivantes est satisfaite :
• m = 0 ou 1,
• R₁ est un groupe -OR₁₁ où R₁₁ est un atome d'hydrogène ou un groupe alkyle en C₁-C₄, linéaire ou ramifié,
• R₂ est un atome d'hydrogène ou un groupe alkyle en C₁-C₆, linéaire ou ramifié, saturé,
• W est une chaîne alkylène ou alkénylène en C₂-C₄, linéaire, non substituée,
• X est un groupe -OR₁₁ où R₁₁ est un atome d'hydrogène ou un groupe alkyle en C₁-C₄, linéaire ou ramifié,
• Y est une chaîne alkylène en C₁₁-C₁₆, ramifiée, non substituée.

11. Composition selon la revendication 10, **caractérisé en ce que** le composé de formule (I') est tel que :
• m = 0 ou 1,
• R₁ est un groupe -OH ou -OCH₃,
• R₂ est un atome d'hydrogène ou un groupe éthyle,
• W est une chaîne triméthylène ou propénylène,
• X est un groupe -OH,
• Y est une chaîne di (C₅-C₇)alkyl éthylène ou di (C₅-C₇)alkyl propylène.

12. Composition selon la revendication 11, **caractérisé en ce que** le composé de formule (I') est tel que : m = 0 ; R₂ est un groupe éthyle ; X = OH ; Y est une chaîne dipentyléthylène ; et W est une chaîne triméthylène.

13. Composition selon l'une quelconque des revendications 7 à 12, **caractérisée en ce que** le composé de formule (I') représente de 0,1 à 2% du poids total de la composition.

14. Composition selon l'une quelconque des revendications 7 à 13, **caractérisée en ce qu'**elle renferme en outre au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes; les agents myorelaxants ; les agents tenseurs ; les agents anti-pollution et/ou anti-radicalaire ; les agents agissant sur la microcirculation ; les agents agissant sur le métabolisme énergétique des cellules ; et leurs mélanges.

15. Utilisation cosmétique d'au moins un composé de formule (I') selon l'une quelconque des revendications 7 à 12, dans une composition adaptée à une application topique sur la peau, comme agent destiné à lisser les rides et ridules.

16. Utilisation selon la revendication 15, **caractérisée en ce que** lesdites rides et ridules sont des rides et ridules d'expression.

17. Procédé de traitement cosmétique d'une peau ridée, comprenant l'application topique sur ladite peau d'une composition selon l'une quelconque des revendications 7 à 14.

18. Procédé selon la revendication 17, **caractérisé en ce que** ladite composition est appliquée sur les zones du visage ou du front marquées par des rides et ridules d'expression et/ou sur les personnes présentant des rides et ridules d'expression.

19. Procédé selon la revendication 17 ou 18, **caractérisé en ce que** ladite composition est appliquée sur les rides et ridules disposées radialement autour de la bouche et/ou des yeux et/ou horizontalement sur le front et/ou situées dans l'espace inter-sourcillier.

## Claims

1. Compound of formula (I) : 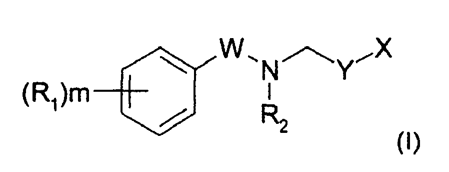 in which:
R₁ denotes a saturated or unsaturated, linear or branched C₁-C₈ alkyl group, or a group -CN, -OR₁₁, -SR₁₁, -NR₁₁R₁₂, -COR₁₁, -COOR₁₁, -CONR₁₁R₁₂, -NR₁₁-CO-R₁₂, -NR₁₁-CO-NR₁₂R₁₃ or -CF₃ or a halogen atom,
where R₁₁, R₁₂ and R₁₃ independently denote a hydrogen atom or a linear or branched C₁-C₄ alkyl group, or an aryl group optionally substituted with a group -OR, -NRR', -COOR or CF₃,
where R and R' independently denote a hydrogen atom or a linear or branched C₁-C₄ alkyl group,
R₂ denotes a hydrogen atom or an unsubstituted, saturated or unsaturated, linear or branched C₁-C₁₂ alkyl group,
W is an unsubstituted, linear C₂-C₄ alkylene or alkenylene chain,
X is a group -OR₁₁ or -NR₁₁R₁₂, where R₁₁ and R₁₂ have the meaning indicated above,
Y denotes a di (C₅-C₇) alkylethylene or di (C₅-C₇) alkylpropylene chain,
m is an integer between 0 and 5,
it being understood that when m is not zero, the groups R₁ may be identical or different,
or its addition salt with an acid or its isomers or its stereoisomers.

2. Compound according to Claim 1, **characterized in that** the salt is obtained by addition with an inorganic acid chosen from hydrochloric, sulphuric, nitric and phosphoric acids.

3. Compound according to Claim 1, **characterized in that** the salt is obtained by addition with an organic acid chosen from succinic, fumaric, lactic, glycolic, citric and tartaric acids.

4. Compound according to any one of Claims 1 to 3, **characterized in that** it is such that at least one of the following conditions is satisfied:
• m = 0 or 1,
• R₁ is a group -OR₁₁ where R₁₁ is a hydrogen atom or a linear or branched C₁-C₄ alkyl group,
• R₂ is a hydrogen atom or a saturated, linear or branched C₁-C₆ alkyl group,
• W is an unsubstituted, linear C₂-C₄ alkylene or alkenylene chain,
• X is a group -OR₁₁ where R₁₁ is a hydrogen atom or a linear or branched C₁-C₄ alkyl group,
• Y is a di(C₅-C₇)alkylethylene chain.

5. Compound according to any one of Claims 1 to 3, **characterized in that**:
• m = 0 or 1,
• R₁ is a group -OH or -OCH₃,
• R₂ is a hydrogen atom or an ethyl group,
• W is a trimethylene or propenylene chain,
• X is a group -OH,
• Y is a di(C₅-C₇)alkylethylene or di(C₅-C₇)alkylpropylene chain.

6. Compound according to Claim 5, **characterized in that** it is such that: m = 0; R₂ is an ethyl group; X = OH; Y is a dipentylethylene chain; and W is a trimethylene chain.

7. Composition, suitable for topical application to the skin, comprising, in a physiologically acceptable medium, at least one compound of formula (I'): 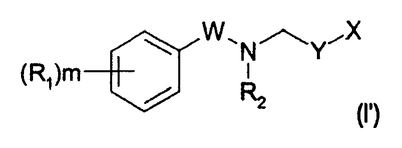 in which:
R₁ denotes a saturated or unsaturated, linear or branched C₁-C₈ alkyl group, or a group -CN, -OR₁₁, -SR₁₁, -NR₁₁R₁₂, -COR₁₁, -COOR₁₁, -CONR₁₁R₁₂, -NR₁₁-CO-R₁₂. -NR₁₁-CO-NR₁₂R₁₃ or -CF₃ or a halogen atom,
where R₁₁, R₁₂ and R₁₃ independently denote a hydrogen atom or a linear or branched C₁-C₄ alkyl group, or an aryl group optionally substituted with a group -OR, -NRR', -COOR or CF₃,
where R and R' independently denote a hydrogen atom or a linear or branched C₁-C₄ alkyl group,
R₂ denotes a hydrogen atom or an unsubstituted, saturated or unsaturated, linear or branched C₁-C₁₂ alkyl group,
W is an unsubstituted, linear C₂-C₄ alkylene or alkenylene chain or a methylene chain,
X is a group -OR₁₁ or -NR₁₁R₁₂, where R₁₁ and R₁₂ have the meaning indicated above,
Y denotes an unsubstituted, linear or branched C₁₁-C₂₀ alkylene or alkenylene chain,
m is an integer between 0 and 5,
it being understood that when m is not zero, the groups R₁ may be identical or different,
or an addition salt with an acid or an isomer or stereoisomer of this compound.

8. Composition according to Claim 7, **characterized in that** the salt is obtained by addition with an inorganic acid chosen from hydrochloric, sulphuric, nitric and phosphoric acids.

9. Composition according to Claim 7, **characterized in that** the salt is obtained by addition with an organic acid chosen from succinic, fumaric, lactic, glycolic, citric and tartaric acids.

10. Composition according to any one of Claims 7 to 9, **characterized in that** the compound of formula (I') is such that at least one of the following conditions is satisfied:
• m = 0 or 1,
• R₁ is a group -OR₁₁ where R₁₁ is a hydrogen atom or a linear or branched C₁-C₄ alkyl group,
• R₂ is a hydrogen atom or a saturated, linear or branched C₁-C₆ alkyl group,
• W is an unsubstituted, linear C₂-C₄ alkylene or alkenylene chain,
• X is a group -OR₁₁ where R₁₁ is a hydrogen atom or a linear or branched C₁-C₄ alkyl group,
• Y is an unsubstituted, branched C₁₁-C₁₆ alkylene chain.

11. Composition according to Claim 10, **characterized in that** the compound of formula (I') is such that:
• m = 0 or 1,
• R₁ is a group -OH or -OCH₃,
• R₂ is a hydrogen atom or an ethyl group,
• W is a trimethylene or propenylene chain,
• X is a group -OH,
• Y is a di(C₅-C₇)alkylethylene or di(C₅-C₇)alkylpropylene chain.

12. Composition according to Claim 11, **characterized in that** the compound of formula (I') is such that: m = 0; R₂ is an ethyl group; X = OH; Y is a dipentylethylene chain; and W is a trimethylene chain.

13. Composition according to any one of Claims 7 to 12, **characterized in that** the compound of formula (I') represents from 0.1 to 2% of the total weight of the composition.

14. Composition according to any one of Claims 7 to 13, **characterized in that** it additionally contains at least one compound chosen from: desquamating agents; moisturizing agents; depigmenting or propigmenting agents; antiglycation agents; NO-synthase inhibitors; agents stimulating the synthesis of dermal or epidermal macromolecules and/or preventing their degradation; agents stimulating the proliferation of fibroblasts and/or of the keratinocytes or stimulating the differentiation of the keratinocytes; muscle-relaxing agents; tightening agents; antipollution and/or anti-radical agents; agents acting on the microcirculation; agents acting on the energy metabolism of the cells; and mixtures thereof.

15. Cosmetic use of at least one compound of formula (I') according to any one of Claims 7 to 12, in a composition suitable for topical application to the skin, as agent intended for smoothing wrinkles and fine lines.

16. Use according to Claim 15, **characterized in that** the said wrinkles and fine lines are expression wrinkles and fine lines.

17. Method for the cosmetic treatment of a wrinkled skin, comprising the topical application to the said skin of a composition according to any one of Claims 7 to 14.

18. Method according to Claim 17, **characterized in that** the said composition is applied to the areas of the face or of the forehead marked by expression wrinkles and fine lines, and/or to people having expression wrinkles and fine lines.

19. Method according to Claim 17 or 18, **characterized in that** the said composition is applied to the wrinkles and fine lines arranged radially around the mouth and/or the eyes and/or horizontally on the forehead and/or situated in the inter-superciliary space.

## Patentansprüche

1. Verbindung der Formel (I): 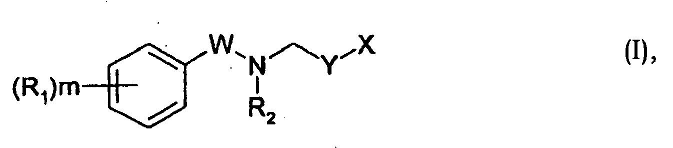 worin bedeuten:
R₁ eine geradkettige oder verzweigte, gesättigte oder ungesättigte C₁₋₈-Alkylgruppe oder eine Gruppe -CN, -OR₁₁, SR₁₁, -NR₁₁R₁₂, -COR₁₁, -COOR₁₁, CONR₁₁R₁₂, -NR₁₁-CO-R₁₂, -NR₁₁-CO-NR₁₂R₁₃ oder -CF₃ oder ein Halogenatom,
wobei R₁₁, R₁₂ und R₁₃ unabhängig voneinander ein Wasserstoffatom oder eine geradkettige oder verzweigte C₁₋₄-Alkylgruppe oder eine gegebenenfalls mit einer Gruppe -OR, -NRR', -COOR oder CF₃ substituierte Arylgruppe bedeuten,
wobei R und R' unabhängig voneinander ein Wasserstoffatom oder eine geradkettige oder verzweigte C₁₋₄-Alkylgruppe bedeuten,
R₂ ein Wasserstoffatom oder eine unsubstituierte, gesättigte oder ungesättigte, geradkettige oder verzweigte Alkylgruppe mit 1 bis 12 Kohlenstoffatomen,
W eine unsubstituierte, geradkettige Alkylen- oder Alkenylengruppe mit 2 bis 4 Kohlenstoffatomen,
X eine Gruppe -OR₁₁ oder -NR₁₁R₁₂, wobei R₁₁ und R₁₂ die oben angegebenen Bedeutungen aufweisen,
Y eine Di(C₅₋₇)alkylethylenkette oder Di(C₅₋₇)alkylpropylenkette, m 0 oder eine ganze Zahl von 1 bis 5,
mit der Maßgabe, dass die Gruppen R₁ gleich oder verschieden sein können, wenn m von 0 verschieden ist,
oder ihre Additionssalze mit einer Säure oder ihre Isomere oder Stereoisomere.

2. Verbindung nach Anspruch 1, **dadurch gekennzeichnet, dass** das Salz durch Zugabe einer anorganischen Säure gebildet wird, die unter Salzsäure, Schwefelsäure, Salpetersäure und Phosphorsäure ausgewählt ist.

3. Verbindung nach Anspruch 1, **dadurch gekennzeichnet, dass** das Salz durch Zugabe einer organischen Säure gebildet wird, die unter Bernsteinsäure, Fumarsäure, Milchsäure, Glycolsäure, Citronensäure und Weinsäure ausgewählt ist.

4. Verbindung nach einem der Ansprüche 1 bis 3, **dadurch gekennzeichnet, dass** es sich um eine Verbindung handelt, die mindestens eine der folgenden Bedingungen erfüllt:
• m = 0 oder 1,
• R₁ bedeutet eine Gruppe -OR₁₁, wobei R₁₁ ein Wasserstoffatom oder eine geradkettige oder verzweigte C₁₋₄-Alkylgruppe bedeutet,
• R₂ ist ein Wasserstoffatom oder eine gesättigte, geradkettige oder verzweigte C₁₋₆-Alkylgruppe,
• W ist eine unsubstituierte, geradkettige Alkylen- oder Alkenylenkette mit 2 bis 4 Kohlenstoffatomen,
• X ist eine Gruppe -OR₁₁, worin R₁₁ ein Wasserstoffatom oder eine geradkettige oder verzweigte C₁₋₄-Alkylgruppe bedeutet,
• Y bedeutet eine Di(C₅₋₇)alkylethylenkette.

5. Verbindung nach einem der Ansprüche 1 bis 3, **dadurch gekennzeichnet, dass**:
• m = 0 oder 1,
• R₁ bedeutet -OH oder -OCH₃,
• R₂ ist ein Wasserstoffatom oder die Ethylgruppe,
• W bedeutet eine Trimethylenkette oder Propenylenkette,
• X ist -OH,
• Y bedeutet eine Di(C₅₋₇)alkylethylenkette oder Di(C₅₋₇)alkylpropylenkette.

6. Verbindung nach Anspruch 5, **dadurch gekennzeichnet, dass**: m = 0; R₂ bedeutet Ethyl; X = OH; Y ist eine Dipentylethylenkette; und W bedeutet eine Trimethylenkette.

7. Zusammensetzung, die für eine topische Anwendung auf die Haut geeignet ist und in einem physiologisch akzeptablen Medium mindestens eine Verbindung der folgenden Formel (I') enthält: 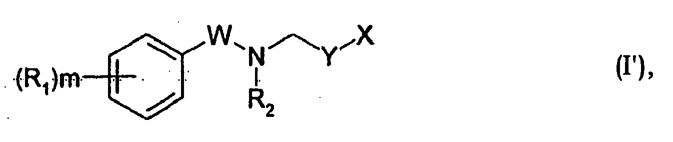 worin bedeuten:
R₁ eine geradkettige oder verzweigte, gesättigte oder ungesättigte C₁₋₈-Alkylgruppe oder eine Gruppe -CN, -OR₁₁, SR₁₁, -NR₁₁R₁₂, -COR₁₁, -COOR₁₁, CONR₁₁R₁₂, -NR₁₁-CO-R₁₂, -NR₁₁-CO-NR₁₂R₁₃ oder -CF₃ oder ein Halogenatom,
wobei R₁₁, R₁₂ und R₁₃ unabhängig voneinander ein Wasserstoffatom oder eine geradkettige oder verzweigte C₁₋₄-Alkylgruppe oder eine gegebenenfalls mit einer Gruppe -OR, -NRR', -COOR oder CF₃ substituierte Arylgruppe bedeuten,
wobei R und R' unabhängig voneinander ein Wasserstoffatom oder eine geradkettige oder verzweigte C₁₋₄-Alkylgruppe bedeuten
R₂ ein Wasserstoffatom oder eine unsubstituierte, gesättigte oder ungesättigte, geradkettige oder verzweigte Alkylgruppe mit 1 bis 12 Kohlenstoffatomen;
W eine unsubstituierte geradkettige Alkylen- oder Alkenylengruppe mit 2 bis 4 Kohlenstoffatomen oder eine Methylenkette, X eine Gruppe -OR₁₁ oder -NR₁₁R₁₂, wobei R₁₁ und R₁₂ die oben angegebenen Bedeutungen aufweisen,
Y eine unsubstituierte, geradkettige oder verzweigte Alkylen- oder Alkenylengruppe mit 11 bis 20 Kohlenstoffatomen,
m 0 oder eine ganze Zahl von 1 bis 5,
mit der Maßgabe, dass die Gruppen R₁ gleich oder verschieden sein können, wenn m von 0 verschieden ist,
oder ihre Additionssalze mit einer Säure oder ihre Isomere oder Stereoisomere.

8. Zusammensetzung nach Anspruch 7, **dadurch gekennzeichnet, dass** das Salz durch Zugabe einer anorganischen Säure gebildet wird, die unter Salzsäure, Schwefelsäure, Salpetersäure und Phosphorsäure ausgewählt ist.

9. Zusammensetzung nach Anspruch 7, **dadurch gekennzeichnet, dass** das Salz durch Zugabe einer organischen Säure gebildet wird, die unter Bernsteinsäure, Fumarsäure, Milchsäure, Glycolsäure, Citronensäure und Weinsäure ausgewählt ist.

10. Zusammensetzung nach einem der Ansprüche 7 bis 9, **dadurch gekennzeichnet, dass** die Verbindung der Formel (I') eine Verbindung ist, die mindestens eine der folgenden Bedingungen erfüllt:
• m = 0 oder 1,
• R₁ bedeutet eine Gruppe -OR₁₁, wobei R₁₁ ein Wasserstoffatom oder eine geradkettige oder verzweigte C₁₋₄-Alkylgruppe bedeutet,
• R₂ ist ein Wasserstoffatom oder eine gesättigte, geradkettige oder verzweigte C₁₋₆-Alkylgruppe,
• W ist eine unsubstituierte, geradkettige Alkylen- oder Alkenylenkette mit 2 bis 4 Kohlenstoffatomen,
• X ist eine Gruppe -OR₁₁, worin R₁₁ ein Wasserstoffatom oder eine geradkettige oder verzweigte C₁₋₄-Alkylgruppe bedeutet,
• Y bedeutet eine unsubstituierte, verzweigte C₁₁₋₁₆-Alkylenkette.

11. Zusammensetzung nach Anspruch 10, **dadurch gekennzeichnet, dass** die Verbindung der Formel (I') eine Verbindung ist, worin:
• m = 0 oder 1,
• R₁ bedeutet -OH oder -OCH₃,
• R₂ ist ein Wasserstoffatom oder die Ethylgruppe,
• W bedeutet eine Trimethylenkette oder Propenylenkette,
• X ist -OH,
• Y bedeutet eine Di(C₅₋₇)alkylethylenkette oder Di(C₅₋₇)alkylpropylenkette.

12. Zusammensetzung nach Anspruch 11, **dadurch gekennzeichnet, dass** die Verbindung der Formel (I') so ist, dass: m = 0; R₂ bedeutet Ethyl; X = OH; Y ist eine Dipentylethylenkette; und W bedeutet eine Trimethylenkette.

13. Zusammensetzung nach einem der Ansprüche 7 bis 12, **dadurch gekennzeichnet, dass** die Verbindung der Formel (I') 0,1 bis 2 % des Gesamtgewichts der Zusammensetzung ausmacht.

14. Zusammensetzung nach einem der Ansprüche 7 bis 13, **dadurch gekennzeichnet, dass** sie ferner mindestens eine Verbindung enthält, die ausgewählt ist unter: abschuppenden Wirkstoffen; Hydratisierungsmitteln; depigmentierenden oder pigmentierungsfördernden Wirkstoffen; Antiglycationswirkstoffen; NO-Synthase-Inhibitoren; Wirkstoffen, die die Synthese von Macromolekülen der Dermis oder Epidermis stimulieren und/oder ihre Zersetzung verhindern; Wirkstoffen, die die Proliferation von Fibroblasten und/oder Keratinocyten stimulieren oder die Differenzierung von Keratinocyten stimulieren; myorelaxierenden Wirkstoffen; straffenden Wirkstoffen; Antipollutionswirkstoffen und/oder Radikalfängern für freie Radikale; Wirkstoffen, die eine Wirkung auf die Mikrozirkulation haben; Wirkstoffen, die auf den Energiehaushalt der Zellen einwirken; und deren Gemischen.

15. Kosmetische Verwendung mindestens einer Verbindung der Formel (I') nach einem der Ansprüche 7 bis 12 in einer Zusammensetzung, die für eine topische Anwendung auf die Haut geeignet ist, als Stoff, der dazu vorgesehen ist, die Falten und Fältchen zu glätten.

16. Verwendung nach Anspruch 15, **dadurch gekennzeichnet, dass** die Falten und Fältchen Mimikfalten und -fältchen sind.

17. Verfahren zur kosmetischen Behandlung von Haut, die Falten aufweist, das das topische Aufbringen eine Zusammensetzung nach einem der Ansprüche 7 bis 14 auf die Haut umfasst.

18. Verfahren nach Anspruch 17, **dadurch gekennzeichnet, dass** die Zusammensetzung auf die Bereiche des Gesichts oder der Stirn, die Mimikfalten und -fältchen aufweisen, und/oder bei Personen aufgetragen wird, die Mimikfalten und -falten aufweisen.

19. Verfahren nach Anspruch 17 oder 18, **dadurch gekennzeichnet, dass** die Zusammensetzung auf Falten und Fältchen aufgebracht wird, die radial um den Mund und/oder die Augen angeordnet sind und/oder horizontal auf der Stirn verlaufen und/oder sich in dem Bereich zwischen den Augenbrauen befinden.
